# EUROPEAN PATENT APPLICATION

(11) **EP 4 600 261 A1**
(43) Date of publication of application: **13.08.2025**
(21) Application number: 23874299.3
(22) Date of filing: 26.09.2023
(51) Int. Cl.: C07K 14/47, C12N 15/12, C12N 15/70, C12N 1/21, C12P 21/02, A61K 38/17, A61P 35/00, A61P 13/08, A61P 29/00, A61P 15/14

(54) **KERATIN YK93-2, AND PREPARATION METHOD THEREFOR, PHARMACEUTICAL COMPOSITION THEREOF AND USE THEREOF**

(30) Priority: 04.10.2022 CN 202211217433
(71) Applicant: INSTITUTE OF MATERIA MEDICA, CHINESE ACADEMY OF MEDICAL SCIENCES, Beijing 100050 (CN)
(72) Inventor: YU, Shishan, Beijing 100050 (CN); CHEN, Xiaoguang, Beijing 100050 (CN); LIU, Yunbao, Beijing 100050 (CN); JIN, Jing, Beijing 100050 (CN); LI, Mi, Beijing 100050 (CN); QU, Jing, Beijing 100050 (CN); LI, Yong, Beijing 100050 (CN); HAO, Mengyao, Beijing 100050 (CN); WANG, Xiaojing, Beijing 100050 (CN); WANG, Mingjin, Beijing 100050 (CN); SHI, Guoru, Beijing 100050 (CN); JIANG, Huimin, Beijing 100050 (CN); MA, Shuanggang, Beijing 100050 (CN); FU, Jiang, Beijing 100050 (CN); LI, Shuying, Beijing 100050 (CN)
(74) Representative: Meissner Bolte Partnerschaft mbB
(86) International application number: PCT/CN2023/121633
(87) International publication number: WO 2024/074107

(57) **Abstract**

The present invention belongs to the field of biopharmaceuticals. Provided are a keratin YK93-2, a nucleic acid molecule encoding the same, an expression vector comprising the nucleic acid molecule, a host cell comprising the expression vector or the genome integrated with the nucleic acid molecule, a preparation method therefor and a pharmaceutical composition thereof. Further provided is the use of the above-mentioned product, such as keratin YK93-2 in the preparation of a drug for treating lung cancer, lymphoma, breast cancer, melanoma, uterine fibroid, prostatic hyperplasia etc.

## Description

### Technical field

The present invention pertains to the field of biopharmaceuticals, relates to a keratin YK93-2, a nucleic acid molecule encoding the keratin YK93-2, an expression vector comprising the nucleic acid molecule, and a host cell comprising the expression vector or the nucleic acid molecule integrated in the genome, and preparation methods of the keratin YK93-2 and pharmaceutical compositions comprising the keratin and use of the keratin and the pharmaceutical composition in the manufacture of medicaments for lactation, analgesia, coagulation or prevention or treatment of prostatic hyperplasia, lung cancer, lymphoma, melanoma, breast cancer, uterine fibroids.

### Background Art

Keratin is a structural protein of the ectodermal cells. According to whether it is fibrosis, it is divided into two categories: soft keratin and hard keratin. Hard keratin is the main component of hair, feathers, hoofs, shells, claws, horns, scales, etc. It is an extremely important structural protein for connective tissue and plays a role in protecting the body.

Because hard keratin is not easy to dissolve in various solvents, and keratin is generally more resistant to protease hydrolysis than other proteins, At present, the study of diagonal protein is more focused on the intracellular soft keratin, however hard keratins which constitute the vast majority keratin resources in nature, that is, hard keratin in the coverings of animal surface (hoof, horns, nails, shells, etc) are rarely studied, not effectively used, and are usually discarded as waste.

With the rapid development of modern biotechnology such as genomics, proteomics, genetic engineering, microbial engineering, etc., the application research of keratin in terms of materials, biology, and medicine has gradually attracted attention. Therefore, it is novel and innovative to prepare and produce hard keratin by utilizing the protein expression system, and further to study its pharmacological effects and develop new drugs with hard keratin as the pharmacodynamic substance.

### Summary of the invention

The technical problem solved by the present invention is to provide a keratin YK 93-2, a nucleic acid molecule encoding keratin YK93-2, an expression vector comprising the nucleic acid molecule, and a host cell comprising the expression vector or the nucleic acid molecule integrated in the genome, and preparation methods of keratin YK93-2, pharmaceutical compositions comprising the keratin YK93-2, and use of the above-mentioned keratin YK93-2, nucleic acid molecule, expression vector, host cell, or pharmaceutical compositions in the manufacture of medicaments for lactation, analgesia, coagulation or prevention or treatment of prostatic hyperplasia, lung cancer, lymphoma, melanoma, breast cancer, uterine fibroids.

In order to solve the technical problems of the present invention, the present invention provides the following technical solutions:
The first aspect of the technical solution of the present invention is to provide a keratin YK93-2, characterized in that the amino acid sequence of the keratin YK93-2 is as follows:
(1) the amino acid sequence shown in SEQ ID NO.1 in the sequence listing; or
(2) the amino acid sequence formed by substitution, deletion or addition of 1-35 amino acids in the amino acid sequence shown in SEQ ID NO.1 in the sequence listing that basically maintains the same biological function.

Further, conventional modification can be performed on keratin YK93-2; or a tag for detection or purification can be attached to keratin YK93-2.

Furthermore, the conventional modification includes acetylation, amidation, cyclization, glycosylation, phosphorylation, alkylation, biotinylation, fluorescent group modification, polyethylene glycol (PEG) modification, immobilization modification, sulfation, oxidation, methylation, deamination, formation of disulfide bonds or disulfide bonds breakage; The tag includes His6, GST, EGFP, MBP, Nus, HA, IgG, FLAG, c-Myc, Profinity eXact.

The second aspect of the technical solution of the present invention provides a nucleic acid molecule encoding the keratin YK93-2 of the first aspect.

Further, the nucleotide sequence of the nucleic acid molecule is:
(1) The nucleotide sequence shown in SEQ ID NO. 2 in the sequence listing.
(2) a nucleotide sequence obtained by sequence optimization based on the nucleotide sequence shown in SEQ ID NO.2.
(3) a nucleotide sequence complementary to the nucleotide sequence in (1) or (2) above-mentioned.

The third aspect of the technical solution of the present invention provides an expression vector, characterized in that the expression vector comprises the nucleic acid molecule described in the second aspect.

Further, the expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K, etc.; the preferred expression vector is the pET series vector; the most preferred expression vector is pET-30a(+).

The fourth aspect of the technical solution of the present invention provides a host cell, characterized in that the host cell comprises the expression vector of the third aspect or the nucleic acid molecule of the second aspect integrated in the genome.

Further, the host cell includes bacteria, yeast, aspergillus, plant cells, or insect cells.

Furthermore, the bacteria include Escherichia coli or yeast.

Competent host cells can be BL21 series, Transetta series, Rosetta series, DH5α series, JM series, Top series, Orgami series, Trans1-T1, TG1, TB1; Y11430, MG1003, GS115 (AOX1), KM71, SMD1168, etc.; The preferred expression competent cells are BL21 (DE3), Transetta (DE3).

The fifth aspect of the technical solution of the present invention provides a method for preparing the keratin YK93-2 of the first aspect, which is characterized in that it comprises the following steps:
A. Synthesizing the nucleic acid molecule corresponding to the keratin YK93-2 described in the first aspect, ligating the nucleic acid molecule into a corresponding expression vector, transforming the expression vector into a host cell, culturing the host cell with the expression vector in fermentation equipment under certain conditions and inducing expression of keratin YK93-2 to obtain a crude protein solution containing keratin YK93-2.
B. Separating, purifying and drying the crude protein solution expressed in step A to obtain keratin YK93-2.

Further, in step A, the host cell is mainly selected from Escherichia coli, the keratin YK93-2 is expressed in inclusion bodies of Escherichia coli, and the fermentation equipment includes shake flask or fermentation tank.

Further, in step A, after inducing the expression of keratin YK93-2, using detergent to wash impurities, using a solution to dissolve to obtain a crude protein solution.

Further, the medium in step A can be LB medium, TB medium, SB medium, SOB medium, SOC medium, PDA medium, YPD medium, rose bengal medium, salt Czapek Dox medium, DOBA medium, rice koji medium and its modified formula, etc.; for shake flask fermentation, LB medium is preferred; for fermentation tank, LB medium and its modified formula are preferred.

Further, the inducer in step A can be IPTG, lactose, arabinose, etc.; preferably IPTG, lactose.

Further, in step A, the obtained fermentation broth is centrifuged to discard the supernatant; The precipitate is suspended in a buffer, followed by bacterial cell disruption and subsequent centrifugation to remove the supernatant; After the precipitate is washed with a detergent, it is then dissolved in a urea solution to obtain a YK93-2 crude protein solution.

Among them, the buffer is preferably buffer A, and its dosage is: fermentation broth volume: buffer A volume=1~100:1, preferably 10:1;
The detergents can be urea solution, guanidine hydrochloride solution, triton and buffer A, etc., preferably urea solution, most preferably 2M urea solution (containing 1% Triton). The dosage is: fermentation broth volume: 1M urea volume (containing 1% Triton) = 0.2~100:1, preferably 1~15:1;

The urea solution is preferably an 8M urea solution, and its dosage is: fermentation broth volume: 8M urea volume=0.2~100: 1, preferably 2~15: 1.

Further, in step B, the method for separating and purifying includes ultrafiltration or microfiltration membrane technology purification method, column chromatography purification method, salting out method, and dialysis method.

Further, in step B, the method for separating and purifying is as follows:
(1) The dialysis method is to purify the crude protein solution obtained in step A by the dialysis method to obtain the target protein YK93-2 solution.

The molecular weight cut-off of the dialysis bag can be 0.5-10kD, the preferred molecular weight cut-off of the dialysis bag is 3.5-10kD, the most preferred molecular weight cut-off of the dialysis bag is 10 kD.

(2) The ultrafiltration and microfiltration method is to purify the crude protein solution obtained in step A by membrane technology such as ultrafiltration membrane or microfiltration membrane to obtain a concentrated solution of the target protein YK93-2.

Preferably, the microfiltration membrane purification is performed twice, the membrane pore size in the first time is 1000~1500nm, and the membrane pore size in the second time is 20~50nm.

(3) The column chromatography method is to pass the crude protein solution obtained in step A through column chromatography, such as various exchange column or exclusion column chromatography, to separate and purify, to obtain the target protein YK93-2.

The preferred exclusion column is dextran gel column, Superdex 30 Increase, Superdex 75 Increase, Superdex 200 Increase, Superose 6 Increase, etc.; The preferred exchange column is an ion exchange resin column: anion exchange resin column, HiTrap Q FF, HiTrap Capto Q ImpRes, Capto Q ImpRes, HiTrap Capto Q, HiTrap DEAE, Toyopearl Q-650M Toyopearl SuperQ-650M, etc.; Cation exchange resin column: HiTrap SP FF, HiTrap Capto SP ImpRes, Capto SP ImpRes, HiTrap Capto SP, Toyopearl SP-650M, Toyopearl Super SP-650M. The most preferred is an anion exchange resin column.

As the eluent, commonly used eluents in the art can be used, such as water, salt solution. The salt solution includes sodium chloride solution, sodium dihydrogen phosphate solution, disodium hydrogen phosphate solution, sodium acetate, acetic acid, and the like.

(4) The salting-out method is to purify the crude protein solution obtained in step A by salting-out method to obtain the target protein YK93-2 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

Further, the target protein YK93-2 solution purified in step B can be freeze-dried or vacuum-dried into dry powder, or the concentrated solution can be directly spray-dried into dry powder.

The sixth aspect of the technical solution of the present invention provides a pharmaceutical composition, characterized in that the pharmaceutical composition comprises the keratin YK93-2 described in the first aspect or the nucleic acid molecule described in the second aspect or the expression vector described in the third aspect or the host cell described in the fourth aspect and a pharmaceutically acceptable carrier or excipient.

The keratin obtained in the above-mentioned steps of the present invention can be freeze-dried or vacuum-dried into dry powder, or the concentrated liquid can be directly spray-dried into dry powder, and then made into various dosage forms.

The present invention relates to a pharmaceutical composition, which comprises any keratin obtained in the above-mentioned steps and a pharmaceutically acceptable carrier.

The present invention also relates to a pharmaceutical composition comprising the keratin of the present invention as an active ingredient and a conventional pharmaceutical excipient or adjuvant. Generally, the keratin of the present invention accounts for 0.1-100.0% of the total weight of the pharmaceutical composition.

The present invention also provides a pharmaceutical composition, which comprises a pharmaceutical effective dose of protein as an active ingredient and a pharmaceutically acceptable carrier.

The pharmaceutical composition of the present invention can be prepared according to methods known in the art. When used for this purpose, if necessary, the protein of the present invention can be combined with one or more solid or liquid pharmaceutical excipients and/or adjuvants to prepare an appropriate administration form or dosage form that can be used as human drugs or Veterinary drugs.

The keratin of the present invention or the pharmaceutical composition comprising the same can be administered in a unit dosage form. The route of administration can be enteral or parenteral, such as oral, intramuscular, subcutaneous, nasal, oral mucosal, ocular, pulmonary, transdermal, vaginal, peritoneal and rectal, etc., oral administration is preferred.

The keratin protein of the present invention or the pharmaceutical composition comprising the same can be administered by injection. Injections include intravenous injection, intramuscular injection, subcutaneous injection, intradermal injection, intraperitoneal injection, and acupoint injection, etc.

The dosage form for administration can be a liquid dosage form, a solid dosage form or a semi-solid dosage form. Liquid dosage forms can be solutions (including true solutions and colloidal solutions), emulsions (including oil-in-water, water-in-oil and double emulsions), suspensions, injections (including water injections, powder injections and infusions), eye drops, nasal drops, lotion and liniment, etc. The solid dosage form can be tablets (including ordinary tablets, enteric-coated tablets, buccal tablets, dispersible tablets, chewable tablets, effervescent tablets, orally disintegrating tablets), capsules (including hard capsules, soft capsules, and enteric-coated capsules), granules, powders, pellets, dripping pills, suppositories, films, patches, air (powder) sprays, sprays, etc.; semi-solid dosage forms can be ointments, gels, pastes, etc.

The keratin of the present invention can be made into ordinary preparations, slow-release preparations, controlled-release preparations, targeted preparations, and various particle delivery systems.

In order to make a unit administration dosage form into a tablet, various excipients known in the art can be widely used, including diluents, binders, wetting agents, disintegrants, lubricants, glidants. The diluent can be starch, dextrin, sucrose, glucose, lactose, mannitol, sorbitol, xylitol, microcrystalline cellulose, calcium sulfate, calcium hydrogen phosphate, calcium carbonate, etc.; the wetting agents can be water, ethanol, isopropanol, etc.; the binder can be starch slurry, dextrin, syrup, honey, glucose solution, microcrystalline cellulose, acacia slurry, gelatin slurry, sodium carboxymethyl cellulose, methyl cellulose, hydroxypropyl methyl cellulose, ethyl cellulose, acrylic resin, carbomer, polyvinylpyrrolidone, polyethylene dipropyl alcohol, etc.; the disintegrant can be dry starch, microcrystalline cellulose, low-substituted hydroxypropyl cellulose, cross-linked polyvinylpyrrolidone, cross-linked sodium carboxymethyl cellulose, sodium carboxymethyl starch, sodium bicarbonate and citric acid, calcium carbonate, polyoxyethylene sorbitol fatty acid ester, dodecyl sodium sulfonate; the lubricant and glidant can be talc, silicon dioxide, stearate, tartaric acid, liquid paraffin, polyethylene glycol, etc.

The tablets can also be further made into coated tablets, such as sugar-coated tablets, film-coated tablets, enteric-coated tablets, or double-layer tablets and multilayer tablets.

In order to make the administration unit into a pill, various carriers known in the field can be widely used. Examples of carriers are, for example, diluents and absorbents, such as glucose, lactose, starch, cocoa butter, hydrogenated vegetable oil, polyvinylpyrrolidone, polyethylene glycol laurate, kaolin, talc, etc.; binders, such as Gum arabic, xanthan gum, gelatin, ethanol, honey, liquid sugar, rice paste or batter, etc.; disintegrants, such as agar powder, dried starch, alginate, sodium lauryl sulfonate, methyl cellulose, ethyl cellulose, etc..

In order to make the administration unit into a suppository, various carriers known in the field can be widely used. Examples of carriers are, for example, polyethylene glycol, lecithin, cocoa butter, higher alcohols, higher alcohol esters, gelatin, semisynthetic glycerides and the like.

In order to make the administration unit into a capsule, the keratin of the present invention as an active ingredient is mixed with the above-mentioned various carriers, and the resulting mixture is placed into hard gelatin capsules or soft capsules. The keratin of the present invention as an active ingredient can also be made into microcapsules, suspended in an aqueous medium to form a suspension, or filled into hard capsules or made into injections for application.

For example, the keratin of the present invention is prepared into injection preparations, such as solutions, suspension solutions, emulsions and freeze-dried powder injections. Such preparations can be aqueous or non-aqueous, and may contain one and/or more pharmacodynamically acceptable carriers, diluents, binders, lubricants, preservatives, surfactants or dispersants. For example, the diluent can be selected from water, ethanol, polyethylene glycol, 1,3-propylene glycol, ethoxylated isostearyl alcohol, polyoxylated isostearyl alcohol, polyoxyethylene sorbitol fatty acid esters and the like. In addition, in order to prepare an isotonic injection, an appropriate amount of sodium chloride, glucose or glycerin can be added to the injection preparation. In addition, conventional auxiliary solvents, buffers, pH adjusters, etc. can also be added. These auxiliary materials are commonly used in this field.

In addition, if necessary, coloring agents, preservatives, flavors, corrigents, sweeteners or other materials can also be added to the pharmaceutical preparations.

In order to achieve the purpose of medication and enhance the therapeutic effect, the keratin or the pharmaceutical composition of the present invention can be administered by any known administration method.

The administration dosage of the keratin pharmaceutical composition of the present invention depends on many factors, such as the nature and severity of the disease to be prevented or treated, the gender, age, weight, personality and individual response of the patient or animal, the route of administration, the frequency of administrations and the purpose of treatment, therefore the therapeutic dose of the present invention can have a wide range of changes. Generally speaking, the dosage of the pharmaceutical ingredients of the present invention is well known to those skilled in the art. Appropriate adjustments can be made according to the actual amount of the drug contained in the final preparation of the keratin composition of the present invention to meet the requirement of the therapeutically effective amount and accomplish the preventive or therapeutic purpose of the present invention. The appropriate daily dosage range of the keratin of the present invention: the dosage of the keratin of the present invention is from 0.01 to 1000 mg/kg body weight, preferably from 5 to 1000 mg/kg body weight, more preferably from 10 to 500 mg/kg body weight, and most preferably from 20 to 300 mg/kg body weight. The above dosage can be administered in a single dosage form or divided into several, such as two, three or four dosage forms for administration, depending on the clinical experience of the administering doctor and the dosage regimens including the use of other treatments. The total dose required for each treatment can be divided into multiple or single doses for administration. The protein or pharmaceutical composition of the present invention can be taken alone, or be used in combination with other therapeutic drugs or symptomatic drugs with adjusting the dose.

The seventh aspect of the technical solution of the present invention provides use of the keratin YK93-2 according to the first aspect or the nucleic acid molecule according to the second aspect or the expression vector according to the third aspect or the host cell according to the fourth aspect or the pharmaceutical composition according to the sixth aspect in the manufacture of medicaments for lactation, analgesia, coagulation or prevention or treatment of prostatic hyperplasia, lung cancer, lymphoma, melanoma, breast cancer, uterine fibroids.

In order to accomplish the purpose of the present invention, the present invention takes the following technical solutions. Specifically, the preparation of keratin YK93-2 of the present invention includes the following steps:
(1) Synthesizing the nucleotide sequence and determining the accuracy of the sequence;

The preferred nucleotide sequence is shown in SEQ ID No. 2.

(2) Transferring the nucleotide sequence into an expression vector;

The expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K etc. The preferred expression vector is the pET series vector; the most preferred expression vector is pET-30a(+).

(3) Transfecting the expression vector into a host cell;

The host cell can be E. coli or yeast; the preferred host cell is E. coli;

Competent cells can be BL21 series, Transetta series, Rosetta series, DH5α series, JM series, Top series, Orgami series, Trans1-T1, TG1, TB1; Y11430, MG1003, GS115 (AOX1), KM71, SMD1168, etc. The preferred competent cells for expression are BL21 (DE3), Transetta (DE3).

(4) Performing fermentation culture of the host cells to induce the expression of the target protein YK93-2 under appropriate conditions;

Fermentation equipment can use shake flasks or fermentation tanks;

The medium can be LB medium, TB medium, SB medium, SOB medium, SOC medium, PDA medium, YPD medium, rose bengal medium, salt Czapek Dox medium, DOBA medium, rice koji culture medium and their improved formulas, etc.; for shake flask fermentation, LB medium is preferred; for fermentation tank, LB medium and its improved formulas are preferred.

The inducer can be IPTG, lactose, arabinose, etc.; The preferred is IPTG, lactose.

(5) Enriching the Target protein YK93-2 product;

Centrifuging the fermentation broth obtained in step (4), and discarding the supernatant; The precipitate is suspended in a buffer, followed by bacterial cell disruption and subsequent centrifugation to remove the supernatant; After the precipitation is washed with detergents, it is dissolved in a urea solution to obtain a YK93-2 crude protein solution.
Among them, the buffer is preferably buffer A, and its dosage is: fermentation broth volume : buffer A volume=1~100 : 1, preferably 10: 1;
The detergent can be urea solution, guanidine hydrochloride solution, Triton, buffer A, etc., preferably urea solution, most preferably 2M urea solution (may contain 1% Triton). The dosage is: fermentation broth volume: 1M urea volume (may contain 1% Triton)=0.2~100: 1, preferably 1~15: 1;

The urea solution is preferably an 8M urea solution. Its dosage is: fermentation broth volume: 8M urea volume=0.2~100:1, preferably 2~15: 1.

(6) Separation and purification of target protein YK93-2.

The crude protein solution obtained in step (5) needs to be purified to obtain the target protein YK93-2. The purification can be carried out by dialysis, or ultrafiltration and microfiltration, or column chromatography, or salting out steps.

A. The dialysis step is to purify the crude protein solution obtained in step (5) by a dialysis method to obtain the target protein YK93-2 solution.

The molecular weight cut-off of the dialysis bag can be 0.5-10 kD, the preferred molecular weight cut-off of the dialysis bag is 3.5-10 kD, and the most preferred molecular weight cut-off of the dialysis bag is 10 kD.

B. The ultrafiltration and microfiltration step is to purify the crude protein solution obtained in step (5) by membrane technology such as ultrafiltration membrane or microfiltration membrane to obtain the target protein YK93-2 concentrated solution.

Preferably, the microfiltration membrane purification is performed twice. The membrane pore size is 1000~1500nm in the first time, and the membrane pore size is 20~50nm in the second time.

C. The column chromatography step is to pass the crude protein solution obtained in step (5) through column chromatography, such as various exchange column or exclusion column chromatography, to separate and purify and to obtain the target protein YK93-2.

The preferred exclusion column is dextran gel column, Superdex 30 Increase, Superdex 75 Increase, Superdex 200 Increase, Superose 6 Increase, etc.; The preferred exchange column is ion exchange resin column: anion exchange resin column, HiTrap Q FF, HiTrap Capto Q ImpRes, Capto Q ImpRes, HiTrap Capto Q, HiTrap DEAE, Toyopearl Q-650M, Toyopearl SuperQ-650M, etc.; Cation exchange resin column, HiTrap SP FF, HiTrap Capto SP ImpRes, Capto SP ImpRes, HiTrap Capto SP, Toyopearl SP-650M, Toyopearl Super SP-650M. The most preferred is anion exchange resin column.

As the eluent, commonly used eluents in the art can be used, such as water, salt solution, the salt solution includes sodium chloride solution, sodium dihydrogen phosphate solution, disodium hydrogen phosphate solution, sodium acetate, acetic acid, and the like.

D. The salting-out step is to purify the crude protein solution obtained in step (5) by a salting-out method to obtain the target protein YK93-2 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

The target protein YK93-2 solution purified from steps A to D can be freeze-dried or vacuum dried into dry powder, or the concentrated solution can be directly spray-dried into dry powder.

The beneficial technical effects of the present invention:
1. The protein of the present invention is a keratin obtained for the first time, and the preparation method of the present invention has the characteristics of high yield and high sample purity.
2. By studying the pharmacodynamics of the protein YK93-2 on the mouse prostatic hyperplasia model, in the invention it proves that the protein YK93-2 can significantly reduce the wet weight of the prostate and the prostate index.
3. By studying the pharmacodynamics of the protein YK93-2 on Lewis lung cancer, in the invention it proves that the protein YK93-2 has a significant effect on inhibiting lung cancer proliferation.
4. By studying the lactation of pregnant mice with protein YK93-2, in the invention it proves that the protein YK93-2 can increase the serum prolactin content of female mice to a certain extent.
5. By studying the pharmacodynamics of the protein YK93-2 on EL-4 lymphoma, in the invention it proves that protein YK93-2 has obvious inhibitory effect on lymphoma proliferation.
6. By studying the pharmacodynamics of the protein YK93-2 on the mouse analgesic model, in the invention it proves that the protein YK93-2 can reduce the number of times of writhing to a certain extent, which has a certain analgesic effect.
7. By studying the pharmacodynamics of protein YK93-2 on B16F10 melanoma, in the invention it proves that protein YK93-2 has obvious inhibiting effect on melanoma proliferation.

### Brief Description of the Drawings

Figure 1: Analysis of expressed protein YK93-2 by reduced SDS polyacrylamide gel electrophoresis (SDS-PAGE).
   (M: Protein molecular weight standard; S: Expressed protein YK93-2)
Figure 2: Effect of protein YK93-2 on the blood routine test of Lewis lung cancer mice.
   (Compared with normal control group, ** P<0.01, *** P<0.001; Compared with the model group, # P<0.05, ## P<0.01, ### P<0.001)
Figure 3: Effect of protein YK93-2 on the lactation yield of female mice.
   (Compared with normal control group, **P<0.01, *** P<0.001; Compared with the model group, # P<0.05, ## P<0.01, ### P<0.001)
Figure 4: Effect of protein YK93-2 on the serum prolactin of the female mice.
   (Compared with normal control group, ** P<0.01, *** P<0.001; Compared with the model group, # P<0.05, ## P<0.01, ### P<0.001)
Figure 5: Effect of protein YK93-2 on the blood routine test of EL-4 lymphoma mice.
   (Compared with normal control group, ** P<0.01, *** P<0.001; Compared with the model group, # P<0.05, ## P<0.01, ### P<0.001).
Figure 6: The inhibitory effect of protein YK93-2 on the acetic acid-induced writhing of mice.
   (Compared with normal control group, ** P<0.01, *** P<0.001; Compared with the model group, # P<0.05, ## P<0.01, ### P<0.001).
Figure 7: The effect of protein YK93-2 on the blood routine test of B16F10 melanoma mice.
   (Compared with normal control group, ** P<0.01, *** P<0.001; Compared with the model group, # P<0.05, ## P<0.01, ### P<0.001).

### EXAMPLES

The following examples and pharmacological activity test examples are used to further illustrate the present invention, but this does not mean any limitation to the present invention.

The experimental methods in the following examples and pharmacological activity test examples are conventional methods unless otherwise specified; the experimental materials used, unless otherwise specified, are purchased from conventional biochemical reagent companies.

### Example 1 Preparation of protein YK93-2 crude solution A by Shake flask fermentation (LB medium)

The nucleotide sequence shown in SEQ ID No.2 was synthesized and transferred it into the pET-30a(+) vector. It was confirmed by sequencing that an expression vector comprising the correct sequence was obtained. And the expression vector was transfected into BL21 (DE3) cells and expression competent host cells comprising the target nucleotide sequence were obtained. The expression competent host cells were added into LB medium and incubated in a shaker at 37°C and 220 rpm for 1 hour to obtain a recombinant strain.

An LBA plate containing Kanamycin was streaked with a dip of the recombinant strain, and placed upside down in a 37°C constant temperature incubator overnight for 16 hours.

400 ml of LB medium was prepared, divided into 2 bottles, 200 ml in each bottle. Kanamycin (final concentration 50 µg/ml) was added to each bottle (200 ml) of LB medium. A single colony on the plate was taken and added to the LB medium and amplified and cultured overnight at 37°C and 220 rpm in the shaker to obtain seed liquid.

9.6 L of LB medium was prepared, divided into 48 bottles, 200 ml in each bottle. Each bottle (200 ml) of LB medium was added with Kanamycin (final concentration 50 µg/ml), then added with 2 ml of the seed liquid, and cultured in a shaker at 37°C and 220 rpm for 2-3 hours. The OD₆₀₀ was monitored, when the OD₆₀₀ reached about 1.0, an inducer was added to induce protein expression in a shaker, and the induction conditions were selected from the following table.

| | Inducer | Induction temperature | Induction time | Shaker speed |
|---|---|---|---|---|
| Inducing conditions | IPTG (Final concentration 0.5mM) | 16°C | 16h | 220 rpm |
| | | 37°C | 5h | |

All bottles of bacteria liquid were combined, centrifuged at 7000 rpm for 5 minutes, and the supernatant was discarded after sterilization. The precipitate was suspended in about 1 L of buffer, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes, the supernatant was discarded, and the precipitate (i.e. inclusion body) was obtained. The precipitate was washed twice with 600 ml of detergent, centrifuged and the supernatant was discarded. The precipitate was dissolved in urea solution 3 times, around 600 ml each time. The three solutions were combined and centrifuged at 7000 rpm for 30 minutes. The precipitate was discarded and the supernatant was the crude protein solution A.

| Buffer | detergent | Urea solution |
|---|---|---|
| buffer A | 1M urea solution (It can contain Triton) | 8M urea solution (It can contain Tris/HCl buffer or NaH₂PO₄/Na₂HPO₄ Buffer) |
| | 2M urea solution (It can contain Triton) | 4M urea solution (It can contain Tris/HCl buffer) |

Protein YK93-2 crude solution A was analyzed by reduced SDS-PAGE, with the separation gel concentration being 12.5%, and then stained with Coomassie brilliant blue R250 method; a clear blue band was shown near the molecular weight of 55KD.

### Example 2 Preparation of protein YK93-2 crude solution B in a fermentation tank

In Example 1, an expression vector was synthesized and sequenced to confirm that the expression vector comprising the sequence shown in SEQ ID No. 2 was obtained. The expression vector was transfected into BL21 (DE3) cells and expression-competent host cells comprising the target nucleotide sequence were obtained. The expression competent host cells were added into LB medium and cultured in a shaker at 37°C and 220 rpm for 1 hour to obtain a recombinant strain.

In the LBA plate containing Kanamycin, 100 µl of the recombinant strain was added, spread with the spreader until it became evenly dry, and the plate was placed upside down in a constant temperature incubator at 37°C for overnight culture. Three single colonies were taken separately, streaked on plates containing Kanamycin, and then the plates were cultured overnight. After the verifications of three batches of shake flask fermentation and expression were confirmed to be correct, the strains were preserved with 15% glycerol and were divided into 1 ml for each to obtain a working cell bank, which was stored in a refrigerator at -80°C for later use.

100 µl of 1 glycerol bacteria stock was taken out from the working cell bank, added with 40 ml of LB medium, added with Kanamycin (final concentration 50 µg/ml), cultured in a shaker at 37°C and 220 rpm for 6 hours to obtain a first-level seed liquid.

1.2ml of the first-level seed liquid was taken, added to 120 ml of LB medium, added with Kanamycin (final concentration 50 µg/ml), and then cultured in a shaker at 37°C and 220 rpm for 7 hours to obtain a second-level seed liquid.

3L of modified LB broth was added to a 5L fermentation tank, then added with 120 ml of the second-level seed liquid and 3 ml of Kanamycin (final concentration 50 µg/ml), and cultured at 37°C and 30% dissolved oxygen (series speed) for about 4 hours. The OD value was monitored to be around 20 and 15g lactose used as an inducer. Induction was performed at 20°C, fed at a rate of 30ml/hour, and cultured at 20°C for 24 hours.

The bacteria liquid was centrifuged at 7000 rpm for 5 minutes and the supernatant was discarded after sterilization. The precipitate was suspended in about 200 mL of buffer A, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes and the supernatant was discarded.

1M urea solution (including 1% Triton) was added to the precipitate and wash it three times, 600 mL each time, centrifuge and the supernatant was discarded. Then 4M urea solution was added to the precipitate and dissolved once and 8M urea solution to dissolve 2 times, 600 ml each time; The three solutions were combined, centrifuged at 7000 rpm for 30 minutes, the precipitate was discarded, and the supernatant was the crude protein solution B.

Protein YK93-2 crude solution B was analyzed by reduced SDS-PAGE with the separation gel concentration being 12.5% and then stained with Coomassie brilliant blue R250 method; a clear blue band was shown near the molecular weight of 55 KD.

### Example 3 Preparation of protein YK93-2 from protein crude solution B by membrane technology

Protein crude solution B obtained in Example 2 was purified by microfiltration membrane technology: firstly a 1500 nm or 1000 nm ceramic membrane core was used for solid-liquid separation; the inner liquid was discarded, and then a 20 nm or 50 nm ceramic membrane core was used on external liquid for repeated microfiltration to remove urea. The inner liquid of the second microfiltration was freeze-dried to obtain the target protein YK93-2.

### Confirmation of the protein YK93-2 structure:

### 1. Reduced SDS-polyacrylamide gel electrophoresis (SDS-PAGE) analysis

Instrument: Protein electrophoresis apparatus (Bio-Rad).
Methods and results: The protein YK93-2 solution was analyzed by reduced SDS-PAGE with the separation gel concentration being 12.5% and stained with Coomassie brilliant blue R250 method. The molecular weight of YK93-2 band was around 55 KD.

### 2. Complete protein sequence analysis based on LC-MS/MS

Main materials: Acetonitrile, formic acid, ammonium bicarbonate, dithiothreitol (DTT), iodoacetamide (IAA), trypsin, chymotrypsin, Glu-C, Pepsin, Elastase;
Main instruments: Nano Liquid Chromatograph (Thermo EastnLC1200), Mass Spectrometer (Thermo Orbitrap Fusion Lumos). Constant temperature incubator (Zhongyi State Science (Beijing) Technology Co., Ltd. DHP-9052).

### Methods and results:

Protein YK93-2 was subject to pre-treatments such as dissolution replacement, reductive alkylation, and various proteolysis to obtain enzyme-cleaved peptides. The solution of enzyme-cleaved peptides was analyzed by liquid chromatography tandem mass spectrometry. Byonic software was used to analyze the data in the original mass spectrometry file. The coverage rate of the appraisal results was 100%, which was confirmed to be consistent with the target sequence of SEQ ID No. 1.

### Example 4 Preparation of protein YK93-2 by purification from protein crude solution A

The protein crude solution A obtained in Example 1 was purified by the following three methods:

### The first method: dialysis

The protein crude solution A was filtered with a 0.45 µm filter membrane, the filtrate was dialyzed with water for more than 72 hours, and the inner solution was freeze-dried to obtain the target protein YK93-2.

| | |
|---|---|
| Dialysis bag | Molecular weight cutoff: 0.5kD, 3.5kD, 5kD, 10kD |

### The second method: salting out

The protein crude solution A was placed in a stirred container for salting out twice. Saturated ammonium sulfate solution was slowly added along the wall to make the final concentration of ammonium sulfate reach 25% or 50%. During the salting-out process, the protein was separated out. After the salting-out was completed, the solution was filtered. Then 400 ml of pure water was added into the precipitate to suspend, and a saturated solution of ammonium sulfate was slowly added along the wall again to make the final concentration of ammonium sulfate reach 25%. The second salting out and filtration were carried out, and the precipitate was the crude protein extract. The crude protein extract was washed three times with water: 200 ml of pure water was added to suspend, stirred, left standing, and filtered. After repeated three times, the precipitate was freeze-dried to obtain the target protein YK93-2.

### The third method: column chromatography

The crude protein solution A was purified separately by anion exchange resin column, such as HiTrap Q FF 16/10, HiTrap Capto Q ImpRes, Capto Q ImpRes, HiTrap Capto Q, HiTrap DEAE, etc. The eluent was a gradient elution of NaCl solution, added with 20mM NaH₂PO₄/Na₂HPO₄ buffer (pH 8.0). The elution fractions were combined according to the results of SDS-PAGE electrophoresis detection. The combined eluate was centrifuged twice at 7000 rpm for 1 hour each time. The supernatant was filtered with a 0.45 µm filter membrane, and the filtrates were combined. The filtrates were concentrated by dialysis with water, the molecular weight cut-off of the dialysis bag was 10kD, and the inner liquid was freeze-dried to obtain the target protein YK93-2.

The product protein YK93-2 obtained by the three methods was confirmed to have the same amino acid sequence as that of the protein prepared in Example 3 through the same structural confirmation method as in Example 3.

### Example 5 Preparation of protein YK93-2 by salting out from protein crude solution B

The crude protein solution B obtained in Example 2 was placed in a stirred container for salting out twice. Saturated ammonium sulfate solution was slowly added along the wall to make the final concentration of ammonium sulfate reach 25%. During the salting-out process, the protein was separated out. After the salting-out was completed, the solution was filtered. 400 ml of pure water was added into the precipitate to suspend, and then a saturated solution of ammonium sulfate was slowly added along the wall again to make the final concentration of ammonium sulfate reach 25%. The second salting out and filtration were carried out, and the precipitate was the crude protein extract. The crude protein extract was washed three times with water: 200 ml of pure water was added to suspend, stirred, left standing, and filtered. After repeated three times, the precipitate was freeze-dried to obtain the target protein YK93-2.

The product protein YK93-2 was confirmed to have the same amino acid sequence as that the protein prepared in Example 3 through the same structural confirmation method as in Example 3.

### Pharmacological test

### Experimental example 1 The pharmacodynamic test of protein YK93-2 (the protein in Example 3) on mouse prostatic hyperplasia model.

Animals: Male Kunming mice, weight: 18-20 grams
Drugs: propionate testosterone injection (Ningbo Second Hormon Factory), finasteride
(Merck Sharp & Dohme, MSD)
Instruments: electronic balance

### Experiment grouping:

### Normal control group;

Model group: Androgen loading Model;
Positive control group: finasteride (1mg/kg) group; finasteride (3 mg/kg) group;
YK93-2 (0.5g/kg) group.

### Method:

Preparation of experimental animals: The experimental animals were adapted to the experimental environment (temperature 22°C±2°C, relative humidity 50%±2%) for 1 day.

Subcutaneous injection of propionate testosterone to replicate mice prostatic hyperplasia model: Mice were randomly grouped 10 in each group before the model being established. Propionate testosterone (0.01mg/kg) in soybean oil was injected subcutaneously, once a day for 30 consecutive days. Mice in the normal control group were injected subcutaneously with an equal volume of sodium carboxymethyl cellulose. Immediately after the model was established, finasteride 1mg/kg, finasteride 3m g/kg, or YK93-2 0.5g/kg were orally administered. Mice were weighed on the next day. After 30 days of administration, mice were weighed, executed. The prostate was excised through a median incision in the lower abdomen, the wet weight was weighed, and the prostate index was calculated by the wet weight of the prostate (mg)/body weight of the mouse (g).

### Data statistics:

The average value, standard deviation and standard error of the wet weight of the prostate and the prostate index in each group were calculated. The data from the groups were compared with each other by T.TEST, and P<0.05 was considered as a significant difference.

### Experiment results:

The results were shown in Table 1.

**Table 1. The effects of YK93-2 on mice's weight, wet weight of the prostate and the prostate index**

| Group | Average weight(g) | wet weight of the prostate (mg) | prostate index |
|---|---|---|---|
| Normal control group | 44.6±3.0 | 28.5±5.5 | 0.67±0.14 |
| Model group | 44.6 ± 1.9 | 79.4±15.3*** | 1.78±0.3*** |
| finasteride group(1mg/kg) | 43.5±3.5 | 31.8±4.8### | 0.75±0.12### |
| finasteride(3mg/kg) | 40.7±3.1## | 29.2±1.4### | 0.72±0.08### |
| YK93-2 group (0.5g/kg) | 40.6±1.7### | 50.0±12.8# | 1.24±0.32## |

| | | | |
|---|---|---|---|
| (Compared with the normal control group; * P<0.05, ** P<0.01, *** P<0.001; compared with the model group, # P<0.05, ## P<0.01, ### P<0.001) | | | |

### Experimental conclusions:

1) Androgen loading method can successfully induce prostatic hyperplasia in mice. The mouse of the model group has a significant increase in the wet weight of the prostate and the prostate index after the model is established. Compared with the normal group, P<0.05, there is a statistical difference.
2) The low dose and high dose of the positive medicine finasteride group can effectively reduce the wet weight of the prostate and the prostate index of the model mouse, and the high dose effect is slightly better. Compared with the model group, P <0.05, there is a statistical difference.
3) Compared with the model group, YK 93-2 obviously reduces the wet weight of the prostate and the prostate index of mice (P<0.05).

### Experimental example 2 The pharmacodynamic test of protein YK93-2 (the protein in Example 3) on Lewis lung cancer model

Animals: Male C57BL/6J mouse
Drugs: Cyclophosphamide for injection (CTX), protein YK93-2
Instruments: electronic balance, Five classified blood cell analyser

### Experiment grouping:

Normal control group;
Positive control group: Cyclophosphamide (100mg/kg);
YK93-2 (0.5g/kg) group.

### Method:

Preparation of experimental animals: The experimental animals were adapted to the experimental environment (temperature 22°C±2°C, relative humidity 50%±2%) for 1 day.

Subcutaneous inoculation of Lewis cell suspension to replicate mouse breast cancer model: The Lewis tumor tissue was ground into a suspension with a glass homogenizer, the tumor suspension was aspirated with a syringe, the number of live cells was calculated, the concentration of the cells was adjusted to 2.6*10⁶/ml with physiological saline, and then the suspension was injected to the left armpit of the mouse with 0.2 ml/mouse. On the next day, the mice were randomly divided into normal control group, cyclophosphamide (100mg/kg) group, and YK93-2 (0.5g/kg) group. Cyclophosphamide was injected intraperitoneally for single time, mice in YK93-2 group were given intragastric administration every day for 10 consecutive days, mice in the normal control group were orally given the equal volume of sodium carboxymethyl cellulose. On the day of the treatment, the weight was measured, 20µl of blood was obtained from the orbital blood and the routine blood analysis was performed. The tumor blocks were stripped, weighed and photographed, and the tumor suppression rate was calculated.

### Statistics:

The tumor suppression rate [(The average weight of the tumor in the normal control group - the average weight of the tumor in the experimental group) / The average weight of the tumor in the normal control group × 100 %] and the average value, standard deviation and standard errors of tumor weight were calculated . The data from the groups were compared with each other by T.TEST, and P<0.05 was considered as a significant difference.

### Experimental results:

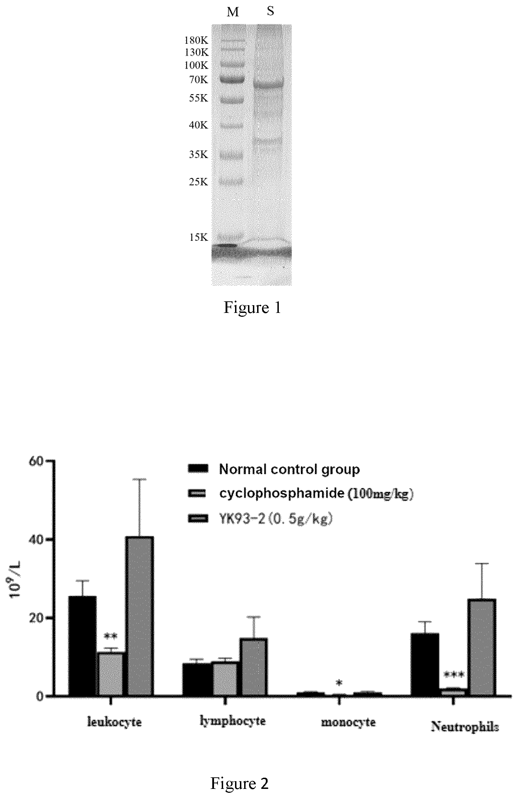

The results were shown in Table 2 and Figure 2.

**Table 2. The inhibitory effects of YK93-2 on mice Lewis lung cancer**

| Group | Dose (g/kg) | Change of weight (g) | | Tumor weight (g) | Tumor suppression (%) |
|---|---|---|---|---|---|
| | | Before administration | After administration | | |
| Normal control group | - | 17.79±0.29 | 23.16±1.04 | 2.99±0.41 | - |
| cyclophosphamide group | 0.1 | 17.60±0.59 | 19.95±0.77*** | 0.14±0.09*** | 95.35 |
| YK93-2 | 0.5 | 17.81±0.43 | 21.83±1.57 | 2.17±0.35*** | 27.38 |

| | | | | | |
|---|---|---|---|---|---|
| (Compared with the normal control group, * P<0.05, ** P<0.01, *** P<0.001) | | | | | |

### Experimental conclusions:

1) Subcutaneous inoculation of Lewis cell can successfully establish mice lung cancer models.
2) The positive drug cyclophosphamide can significantly inhibit the growth of tumor, and the tumor weight is significantly reduced. The tumor suppression rate is up to 95.35%. Compared with the normal control group, P <0.05, there is statistical difference.
3) Compared with the normal control group, YK93-2 (0.5g/kg) can significantly inhibit the growth of lung cancer, the tumor weight is significantly reduced, and the tumor suppression rate is up to 27.38 %. Compared with the normal control group, P <0.05, there is statistical difference. But YK93-2 doesn't reduce the number of leukocytes in peripheral blood.

### Experimental example 3 The pharmacodynamic test of protein YK93-2 (the protein in Example 3) on lactation of pregnant mice

Animals: female Kunming pregnant mice, weight: 30-50 grams
Drug: YK93-2
Instruments: electronic balance

### Experiment grouping:

Normal control group;
Protein YK93-2, (2g/kg) group.

### Method:

Preparation of experimental animals: The experimental animals were adapted to the experimental environment (temperature 22°C±2°C, relative humidity 50%±2%) for 1 day. The parturition date of each pregnant mouse was recorded and the mice were randomly divided into normal control group and YK 93-2 group based on the parturition date.

YK93-2 (2g/kg) was administered by gavage, and the weight and weight gain of the young mice were recorded daily. After 13 days of administration, the female mouse was euthanized using cervical dislocation method, and 400 µl of blood samples were collected from the eye socket, left standing at room temperature for 1-2 hours, centrifuged 1000g for 20 minutes. The content of prolactin in serum was measured.

### Statistics:

The average daily weight gain of young mice (lactation yield of female mice) and serum prolactin content of female mice in each group were calculated. The data from the groups were compared with each other by T.TEST, and P<0.05 was considered as a significant difference.

### Experimental results:

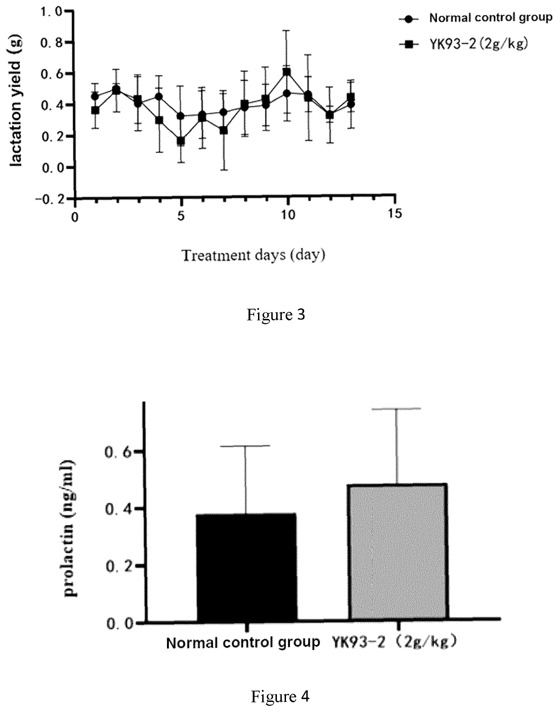

see Figure 3 and Figure 4.

### Experimental conclusion:

1) YK93-2 has no significant effect on the weight gain of young mice.
2) Compared with the normal control group, YK93-2 can to some extent increase the serum prolactin content in female mouse.

### Experimental example 4 The pharmacodynamic test of protein YK93-2 (the protein in Example 3) on EL-4 lymphoma model

Animals: Male C57BL/6J mice
Drugs: Cyclophosphamide for injection (CTX), YK93-2
Instruments: electronic balance, Five classified blood cell analyser

### Experiment grouping:

Normal control group;
Positive control group: cyclophosphamide (100mg/kg);
Protein YK93-2, (2g/kg) group.

### Method:

Preparation of experimental animals: The experimental animals were adapted to the experimental environment (temperature 22°C±2°C, relative humidity 50%±2%) for 1 day.

Subcutaneous inoculation of EL-4 cell suspension to replicate a mouse lymphoma model: The EL-4 tumor tissue was ground into a suspension using a glass homogenizer, the tumor suspension was aspirated with a syringe, the number of live cells was calculated, the cell concentration was adjusted to 2.0*10⁶/ml with physiological saline, and then the suspension was injected into the left armpit of mice with 0.2 ml/mouse. On the next day, mice were randomly divided into a normal control group, a cyclophosphamide (100 mg/kg) group, and a YK93-2 (2g/kg) group. Cyclophosphamide was injected intraperitoneally for single time, mice in the YK93-2 (2g/kg) group were orally administered by gavage daily for 13 consecutive days, while mice in the normal control group were administered orally with an equal volume of sodium carboxymethyl cellulose. On the day of treatment, weight was measured, 20µl of blood was obtained from the orbital blood and the routine blood analysis was performed. The tumor blocks were stripped, weighed and photographed, and the tumor suppression rate was calculated.

### Statistics:

The tumor suppression rate [(The average weight of the tumor in the normal control group - the average weight of the tumor in the experimental group) / The average weight of the tumor in the normal control group × 100 %] and average value, standard deviation and standard errors of tumor weight were calculated. The data from the groups were compared with each other by T.TEST, and P<0.05 was considered as a significant difference.

### Experimental results:

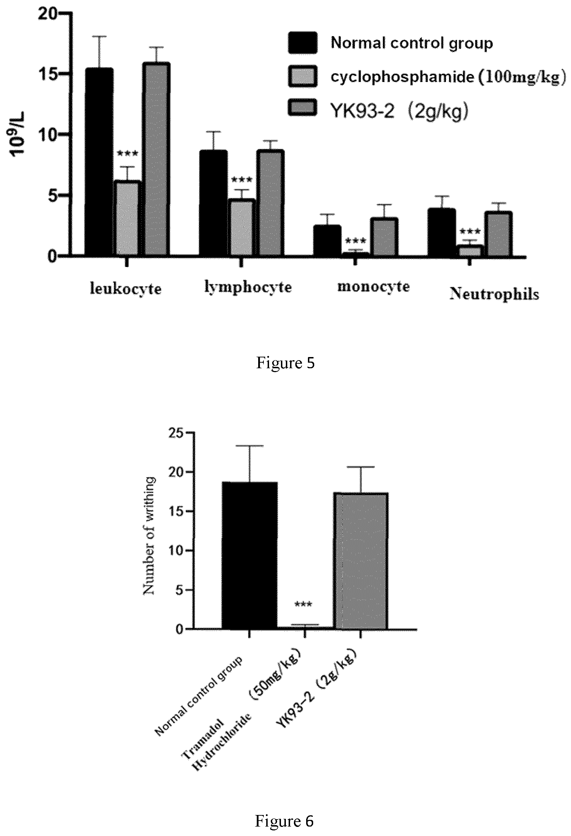

The results were shown in Table 3 and Figure 5.

**Table 3. The inhibitory effects of YK93-2 on mice EL-4 lymphoma**

| Group | Dose (g/kg) | Tumor weight(g) | Tumor suppression (%) |
|---|---|---|---|
| Normal control group | - | 2.81±0.62 | - |
| Cyclophosphamide group | 0.1 | 0.12±0.03*** | 95.67 |
| YK93-2 group | 2 | 2.09±0.34* | 25.59 |

| | | | |
|---|---|---|---|
| (Compared with the normal control group, * P<0.05, ** P<0.01, *** P<0.001) | | | |

### Experimental conclusion:

1) Subcutaneous inoculation of EL-4 cells can establish a mouse T-cell lymphoma model.
2) In the positive drug cyclophosphamide group, tumor growth can be significantly inhibited, tumor weight is significantly reduced, and a tumor inhibition rate of 95.67% is achieved. Compared with the normal control group, P<0.05, there is a statistical difference. In the cyclophosphamide group, leukocytes, lymphocytes, monocytes, and neutrophils can also be significantly reduced, with a statistical difference of P<0.05.
3) Compared with the normal control group, YK93-2 inhibits the growth of lymphoma, significantly reduces tumor weight, and achieves a tumor inhibition rate of 25.59%. Compared with the normal control group, there is a statistical difference of P<0.05. YK93-2 has no significant effect on peripheral blood leukocytes.

### Experimental example 5 The pharmacodynamic test of protein YK93-2 (the protein in Example 3) on mouse pain model

Animals: Male Kunming mice;
Drugs: Tramadol Hydrochloride Sustained Release Tablets, Glacial acetic acid, YK93-2;
Instruments: electronic balance, timer

### Experiment grouping:

Normal control group;
Positive control group: Tramadol Hydrochloride group;
Protein YK93-2, (2g/kg) group.

### Method:

Preparation of experimental animals: The experimental animals were adapted to the experimental environment (temperature 22°C±2°C, relative humidity 50%±2%) for 1 day.

Mice in the YK93-2 (2g/kg) group were administered by gavage, while mice in the model group were given an equal volume of CMC-Na once a day for 7 consecutive days. 30 minutes after the last administration, 1% acetic acid was administered intraperitoneally, and the writhing times of mice within 15 minutes was recorded, and the inhibition rate of drug-induced writhing reaction was calculated. The observation criteria were concave abdominal cavity, extended hind limbs, and raised buttocks.

### Statistics:

The inhibition rate was calculated by (Mean number of writhing reactions in the normal control group - Mean number of writhing reactions in the treatment group)/Mean number of writhing reactions in the normal control group * 100%. The mean value, standard deviation, and standard error of the number of writhing was recorded, and the data from the groups were compared with each other by T.TEST, and P<0.05 was considered as a significant difference.

### Experimental results:

The results were shown in Table 4 and Figure 6.

**Table 4. The inhibitory effects of YK93-2 on pain model in mice**

| Group | Dose | Number of animals (Start/End) | Number of writhing |
|---|---|---|---|
| Normal control group | - | 8/8 | 18.75±13.11 |
| Tramadol Hydrochloride group | 50mg/kg | 8/8 | 0.375±0.74** |
| YK93-2 group | 2g/kg | 8/8 | 17.5±9.15 |

| | | | |
|---|---|---|---|
| (Compared with the normal control group, * P<0.05, ** P<0.01, *** P<0.001) | | | |

### Experimental conclusion:

1) Intraperitoneal injection of 1% acetic acid can successfully induce a mouse pain model. The average number of writhing within 15 minutes is 19 times.
2) In the positive drug tramadol hydrochloride group, pain can be significantly alleviated and the frequency of writhing is significantly reduced, with a statistical difference of P<0.05 compared to the normal control group.
3) Compared with the normal control group, YK93-2 can effectively alleviate pain to a certain extent and reduce the frequency of writhing.

### Experimental example 6 The pharmacodynamic test of protein YK93-2

### (the protein in Example 3) on B16F10 melanoma model

Animals: female C57BL/6J mice;
Drugs: Cyclophosphamide for injection (CTX), YK93-2
Instruments: electronic balance, Five classified blood cell analyser

### Experiment grouping:

Normal control group;
Positive control group: cyclophosphamide group (100mg/kg);
Protein YK93-2, (2g/kg) group.

### Method:

Preparation of experimental animals: The experimental animals were adapted to the experimental environment (temperature 22°C±2°C, relative humidity 50%±2%) for 1 day.

Subcutaneous inoculation of B16F10 cell suspension to replicate a mouse melanoma model: Under sterile conditions, the tumor block was removed, ground into tumor fluid, the concentration was adjusted with physiological saline to 2.58 × 10⁶ /ml, and 0.2 ml was taken to evenly inoculate subcutaneously on the armpit back of mice. On the next day, the animals were randomly grouped and administration began (recorded as Day 0). CTX (100mg/kg) was administered once a week, YK-93-2 (2g/kg) was administered daily, and mice in the normal control group were orally administered an equal volume of sodium carboxymethyl cellulose. On the day of treatment, weight was measured, 20µL of blood was obtained by taking blood from the eye socket and the blood routine analysis was performed, the tumor block was peeled off, weighed and photographed, and the tumor suppression rate was calculated.

### Statistics:

The tumor suppression rate [(The average weight of the tumor in the normal control group - the average weight of the tumor in the experimental group) / The average weight of the tumor in the normal control group × 100 %] and average value, standard deviation and standard errors of tumor weight were calculated. The data from the groups were compared with each other by T.TEST, and P<0.05 was considered as a significant difference.

### Experimental results:

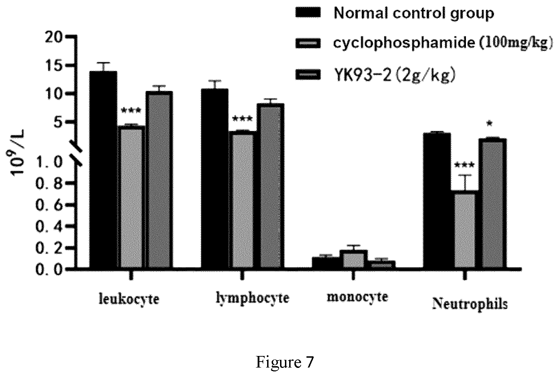

The results were shown in Table 5 and Figure 7.

**Table 5. The inhibitory effects of YK93-2 on mice B16F10 melanoma**

| Group | Dose (g/kg) | Change of weight (g) | | Tumor weight(g) | Tumor suppression (%) |
|---|---|---|---|---|---|
| | | Before administration | After administration | | |
| Normal control group | - | 19.18±0.46 | 20.47±0.50 | 1.81±0.41 | - |
| Cyclophosphamide group | 0.1 | 18.97±0.55 | 19.07±1.46*** | 0.26±0.11*** | 85.82 |
| YK93-2 | 2 | 19.05±0.55 | 19.06±1.46 | 0.68±0.28*** | 62.43 |

| | | | | | |
|---|---|---|---|---|---|
| (Compared with the normal control group, * P<0.05, ** P<0.01, *** P<0.001) | | | | | |

### Experimental conclusion:

1) Subcutaneous inoculation of B16F10 cells can successfully establish a mouse melanoma model.
2) In the positive drug cyclophosphamide group, tumor growth can be significantly inhibited, tumor weight is significantly reduced, and a tumor inhibition rate of 85.82% is achieved. Compared with the normal control group, there is a statistical difference of P<0.05.
3) Compared with the normal control group, YK93-2 (2g/kg) can significantly inhibit the growth of lung cancer, significantly reduce tumor weight, and achieve a tumor inhibition rate of 62.43%. Compared with the normal control group, there is a statistical difference of P<0.05. YK93-2 significantly reduces the number of neutrophils in animal peripheral blood, with a statistical difference of P<0.05.

## Claims

1. A keratin YK93-2, **characterized in that** the amino acid sequence of the keratin YK93-2 is:
(1) the amino acid sequence set forth in SEQ ID NO.1 in the sequence listing; or
(2) an amino acid sequence formed by substitution, deletion or addition of 1-35 amino acids in the amino acid sequence set forth in SEQ ID NO.1 in the sequence listing that basically maintains the same biological function.

2. The keratin YK93-2 according to claim 1, **characterized in that** the keratin YK93-2 can comprise a conventional modification; or be linked with a tag for detection or purification.

3. The keratin YK93-2 according to claim 2, **characterized in that** the conventional modification comprises acetylation, amidation, cyclization, glycosylation, phosphorylation, alkylation, biotinylation, fluorescent group modification, polyethylene glycol (PEG) modification, immobilization modification, sulfation, oxidation, methylation, deamination, formation of disulfide bond(s) or breakage of disulfide bond(s); the tag comprises His6, GST, EGFP, MBP, Nus, HA, IgG, FLAG, c-Myc, Profinity eXact.

4. A nucleic acid molecule encoding the keratin YK93-2 of any one of claims 1 to 3.

5. The nucleic acid molecule according to claim 4, **characterized in that** the nucleotide sequence of the nucleic acid molecule is:
(1) the nucleotide sequence set forth in SEQ ID NO. 2 in the sequence listing;
(2) a nucleotide sequence obtained by sequence optimization based on the nucleotide sequence set forth in SEQ ID NO.2; or
(3) a nucleotide sequence complementary to the nucleotide sequence in (1) or (2) above-mentioned.

6. An expression vector, **characterized in that** the expression vector comprises the nucleic acid molecule of any one of claims 4-5.

7. A host cell, **characterized in that** the host cell comprises the expression vector of claim 6 or the nucleic acid molecule of any one of claims 4-5 integrated in the genome.

8. The host cell according to claim 7, **characterized in that** the host cell comprises bacteria, yeast, Aspergillus, plant cell, or insect cell.

9. The host cell according to claim 8, **characterized in that** the bacteria comprises Escherichia coli or yeast.

10. A method for preparing the keratin YK93-2 according to any one of claims 1 to 3, **characterized in that** it comprises the following steps:
A. synthesizing a nucleic acid molecule corresponding to the keratin YK93-2 according to any one of claims 1 to 3, ligating the nucleic acid molecule to a corresponding expression vector, transforming the expression vector into a host cell, culturing the host cell with the expression vector in a fermentation equipment under certain conditions and inducing the expression of keratin YK93-2 to obtain a crude protein solution comprising keratin YK93-2;
B. separating, purifying and drying the crude protein solution expressed in step A to obtain keratin YK93-2.

11. The method according to claim 10, **characterized in that**, in step A, the host cell is mainly selected from Escherichia coli, the keratin YK93-2 is expressed in inclusion bodies of Escherichia coli, and the fermentation equipment includes shake flask or fermentation tank.

12. The method according to claim 10, **characterized in that**, in step A, after inducing the expression of keratin YK93-2, removing impurities by using a detergent, dissolving keratin YK93-2 in a solution to obtain a crude protein solution.

13. The method according to claim 10, **characterized in that**, in step B, the method of separating and purifying comprises ultrafiltration or microfiltration membrane technology purification method, column chromatography purification method, salting out method, and dialysis method.

14. A pharmaceutical composition, **characterized in that** the pharmaceutical composition comprises the keratin YK93-2 according to any one of claims 1 to 3 and a pharmaceutically acceptable carrier or excipient.

15. Use of the keratin YK93-2 according to any one of claims 1 to 3 or the nucleic acid molecule according to any one of claims 4-5 or the expression vector according to claims 6 or the host cell according to any one of claims 7-9 or the pharmaceutical composition according to claim 14 in the manufacture of a medicament for lactation, analgesia, coagulation or prevention or treatment of prostatic hyperplasia, lung cancer, lymphoma, melanoma, breast cancer, uterine fibroids.
